# FASCICULE DE BREVET EUROPEEN

(11) **EP 3 795 648 B1**
(45) Date de publication et mention de la délivrance du brevet: **03.04.2024**
(21) Numéro de dépôt: 20204684.3
(22) Date de dépôt: 28.01.2008
(51) Int. Cl.: C09D 9/00, C07C 315/06, C08K 5/053, C11D 7/50, C07C 317/04

(54) **FORMULATION DU DIMÉTHYLSULFOXYDE EN MÉLANGE AVEC UN ADDITIF PERMETTANT D'ABAISSER LE POINT DE CRISTALLISATION DE CE DERNIER, ET APPLICATIONS DE CE MÉLANGE**
FORMULIERUNG VON DIMETHYLSULFOXID IN EINER MISCHUNG MIT EINEM ZUSATZSTOFF ZUR SENKUNG DES KRISTALLISATIONSPUNKTES DES LETZTEREN, UND ANWENDUNGEN DIESER MISCHUNG
FORMULATION OF DIMETHYL SULFOXIDE IN MIXTURE WITH AN ADDITIVE CAPABLE OF REDUCING THE CRYSTALLISATION POINT OF THE LATTER, AND USES OF SAID MIXTURE

(30) Priorité: 05.02.2007 FR 0753055
(43) Date de publication de la demande: 24.03.2021
(62) Demande divisionnaire de: 08761992.0
(73) Titulaire: Gaylord Chemical Company LLC, Slidell LA 70458 (US)
(72) Inventeur: LALLIER, Jean-Pierre, 60610 Lacroix St Ouen (FR); LE VILAIN, Fabienne, 69360 Solaize (FR)
(74) Mandataire: Ter Meer Steinmeister & Partner

(56) Documents cités:
- WO-A1-2006/062534
- GB-A- 2 299 812
- DATABASE WPI Week 200382 Thomson Scientific, London, GB; AN 2003-881361 XP002802020, & JP 2003 105330 A (KURIHARA T) 9 avril 2003 (2003-04-09)
- DATABASE WPI Week 198834 Thomson Scientific, London, GB; AN 1988-237372 XP002802021, & JP S63 163457 A (ASAHI CHEM IND CO LTD) 6 juillet 1988 (1988-07-06)

## Description

La présente invention concerne le diméthylsulfoxyde et ses diverses applications. Elle porte sur l'utilisation d'un mélange de diméthylsulfoxyde (DMSO) avec un additif permettant d'abaisser le point de cristallisation de celui-ci sans entraver son efficacité pour les applications habituelles de ce dernier, telles que, mais sans y être limitées, le décapage des peintures et le nettoyage des surfaces et des graffitis. Elle porte également sur des compositions décapantes et nettoyantes comprenant un tel mélange.

Le DMSO, solvant très polaire, est particulièrement adapté à la formulation de décapants de peintures généralement en association avec un cosolvant moyennement polaire, tel qu'un éther, une cétone, un ester, etc. qui permet de conserver l'efficacité du DMSO en diminuant le coût du mélange. Certaines associations conduisent à de véritables synergies qui, en fonction du type de peinture et de son degré de vieillissement, sont équivalentes à ou se rapprochent de l'efficacité de la combinaison classique : chlorure de méthylène + méthanol (Double Liaison, Physique et Chimie des Peintures et Adhésifs - N° 467-468 - 1995 « Nouveaux décapants à base de DMSO^{®} », J.P. Lallier. On peut noter que les solvants protiques, c'est-à-dire comportant une fonction organique X-H, X pouvant être O, N ou S, comme l'eau et les alcools, ne sont pas indiqués comme co-solvants.

Cependant, un inconvénient du DMSO est son trop haut point de cristallisation, égal à 18°C, ce qui conduit à des problèmes de stockage, notamment à l'extérieur en hiver et à des problèmes de manipulation à basse température.

Il est connu que l'eau permet d'abaisser le point de cristallisation du DMSO : un mélange DMSO/eau 92,5/7,5 (p/p), possède un point de cristallisation de 0°C. Cependant, l'ajout d'eau dans une formule à base de DMSO entraîne un certain nombre de problèmes relatifs à la formulation et à l'application :
- lors de la formulation, l'eau s'associe très fortement au DMSO, engendrant une phase très hydrophile et polaire, et il survient très fréquemment une démixtion lors de l'ajout du cosolvant qui est, quant à lui, non protique et moins polaire ;
- en présence d'eau, le DMSO conduit souvent à une perte d'efficacité, les associations DMSO-eau conduisant à des agrégats moléculaires à faible vitesse de diffusion ;
- la présence d'eau entraîne souvent des problèmes d'épaississement, en particulier avec les épaississants cellulosiques.

Certains mono-alcools ont également une bonne aptitude à faire diminuer le point de cristallisation du DMSO. D'une manière générale, ces alcools d'origine pétrochimique sont étiquetés irritants, nocifs ou toxiques et apportent de l'inflammabilité, ce qui est néfaste par rapport au DMSO dépourvu de tout étiquetage.

Actuellement, les formulateurs sont donc dans l'obligation d'utiliser une installation spéciale permettant de réchauffer les fûts de DMSO ou de les stocker et de les utiliser dans des locaux chauffés.

La Société déposante a cherché à résoudre ce problème, et elle a découvert que l'addition de glycérol à une teneur inférieure à la teneur d'un cosolvant dans le DMSO, laquelle est habituellement de 50 à 90 parties en poids de cosolvant dans un mélange DMSO + cosolvant, permettait d'abaisser très efficacement le point de cristallisation du DMSO. Ainsi, un mélange DMSO + glycérol 80/20 (p/p) présente un point de cristallisation de 0°C.

De plus, et de manière inattendue, cet ajout de de triol permet l'obtention d'un mélange miscible avec les cosolvants habituels, moins polaires, et n'entrave pas l'efficacité du décapage du DMSO formulé avec le triol, contrairement au cas où le DMSO est formulé avec l'eau.

La présente invention repose donc sur le principe :
- d'associer au DMSO le glycérol, un composé organique qui est pourvu de plusieurs groupes hydroxy, favorisant l'abaissement du point de cristallisation du DMSO, et qui est à base de carbone, permettant d'avoir une bonne miscibilité avec les autres constituants organiques traditionnels tels que solvants et activateurs, des compositions décapantes et nettoyantes, évitant des démixtions par reprise d'humidité (ce qui arrive avec l'eau) ; et
- de réaliser cette association avec une teneur suffisamment élevée du triol pour qu'il y ait un abaissement efficace du point de cristallisation sans toutefois arriver aux teneurs d'un cosolvant, fonction pour laquelle un alcool est déconseillé car il nuit à l'efficacité du décapage.

Criobiology (1978), 15(1), 93-108, Boutron P et Kaufmann A cite un mélange DMSO + glycérol (10/1 en poids) meilleur que du DMSO pur pour éviter la cristallisation d'un eutectique lors d'un lent refroidissement. Cette publication ne mentionne pas que ce mélange est intéressant pour abaisser le point de cristallisation du DMSO.

WO 03/048393 A1 (PCT/IT 01/00611) décrit l'utilisation de mélanges DMSO + glycérol dans une toute autre application, à savoir la préparation de solutions tampons pour l'amplification en chaîne par polymérase de l'ADN.

JP 1986-308848 décrit, pour l'industrie microélectronique, comme agent de pelage de photorésists (« photoresist strippers ») un mélange DMSO + propylène glycol utilisé à 100°C. Les formules d'agents de pelage de photorésists sont souvent des mélanges à base d'un solvant dipolaire aprotique tel que la N-méthylpyrrolidone ou le DMSO et d'un éther de glycol. Ce dernier n'a pas la fonction d'abaisser le point de cristallisation du solvant actif lorsqu'il s'agit du DMSO. En particulier, les brevets US 4 401 748, US 4 401 747 et US 4 395 479 citent des mélanges à base de NMP et de diéthylène glycol méthyl éther. Le brevet JP 01042653 cite des mélanges à base DMSO et de diéthylèneglycol monoalkyl éther.

US-5 798 323 et US 2001/0034313 A1 décrivent des compositions de pelage et de nettoyage de photorésists à base d'un solvant et d'une alcanolamine. ; dans l'énumération des solvants possibles, on trouve le DMSO, l'éthylène glycol et le propylène glycol.

JP 2003 105330 A divulgue un agent de stockage frigorifique à base de DMSO et des solvants qui sont utilisés pour diminuer le point de cristallisation du DMSO.

La présente invention a donc d'abord pour objet l'utilisation du glycérol comme additif au diméthylsulfoxyde (DMSO) pour abaisser le point de cristallisation de ce dernier. Le glycérol est un additif « vert », n'entraînant aucun étiquetage pour l'association DMSO + glycérol, le DMSO étant déjà sans étiquetage.

Au triol de l'eau peut être ajoutée, auquel cas du chlorure de sodium peut également être ajouté.

Le mélange glycérol + eau (90/10 p/p) introduit à 20 parties en poids dans le DMSO (mélange pondéral DMSO + glycérol + eau 80/18/2) permet un abaissement du point de cristallisation à -2°C. Dans ce cas, la teneur en eau est de 2% en poids seulement, ce qui n'entrave aucunement les propriétés de miscibilité et d'efficacité du décapage ou du nettoyage dans le cas des compositions décapantes ou nettoyantes. Pour atteindre ce point de cristallisation en utilisant de l'eau pure, il faut une teneur en eau de 8,5% en poids pouvant engendrer des problèmes de démixtion dans certains cas et une inhibition de l'efficacité du décapage ou du nettoyage.

Dans le cas de la présente invention, la quantité d'eau introduite sera donc inférieure à celle engendrant les phénomènes de démixtion en présence d'un cosolvant et d'inhibition de l'efficacité.

On peut aussi utiliser des mélanges DMSO + glycérol + eau et DMSO + glycérol + eau + chlorure de sodium, en utilisant si on le souhaite des mélanges respectivement eau + glycérol ou glycérol + chlorure de sodium issus de fabrications industrielles (par exemple eaux glycérineuses industrielles constituées aux environs de 80% en poids de glycérol + 10% en poids d'eau + 10% en poids de chlorure de sodium). Le chlorure de sodium a pour propriété de retarder encore la cristallisation.

L'additif au DMSO peut ainsi être constitué, pour 100 parties en poids, par
(A) 75 à 100 parties en poids de glycérol;
(B) 0 à 15 parties en poids d'eau ; et
(C) 0 à 10 parties en poids de chlorure de sodium, lequel ne peut être présent que si de l'eau est présente.

Par ailleurs, l'additif constitué par le triol avec éventuellement l'eau et le cas échéant le chlorure peut représenter 5 à 40 parties en poids, en particulier 10 à 30 parties en poids, pour 100 parties en poids de DMSO additivé.

Les Exemples suivants illustrent la présente invention sans toutefois en limiter la portée. Dans les exemples, on a utilisé les abréviations suivantes :
DMSO : diméthylsulfoxyde
MEK : méthyl éthyl cétone

### Exemple 1 : Préparation d'un diméthylsulfoxyde glycérolé

On a préparé une composition chimique ayant la formulation suivante, pour 100 parties en poids :
- 90 parties en poids de DMSO ; et
- 10 parties en poids de glycérol.

Le point de cristallisation de cette composition se situe aux alentours de 10°C. Cette valeur peut être comparée au point de cristallisation du DMSO pur qui est de +18°C.

### Essais de décapage d'une peinture :

On a utilisé cette formulation pour imbiber à température ambiante un morceau de coton déposé sur une plaque de bois (contreplaqué) revêtue d'une peinture glycérophtalique. Après 30 minutes de contact, on a retiré le coton et on a observé la plaque à cet emplacement.

On a conduit le même essai de décapage d'une part avec du DMSO pur et d'autre part avec une formulation à 90 parties en poids de DMSO et 10 parties en poids d'eau.

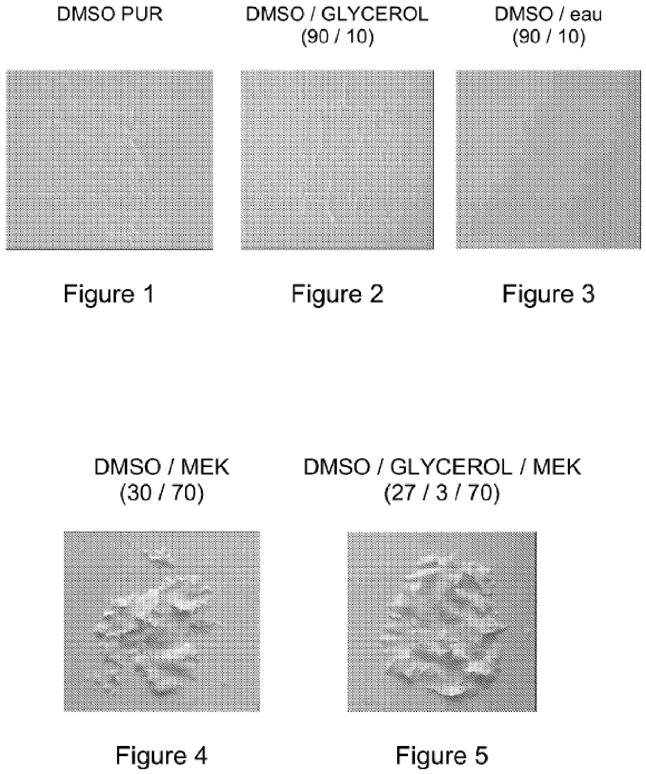

Les résultats visuels de ces trois essais peuvent être observés sur les Figures 1 à 3. On peut voir que, si l'eau a un effet d'inhibition du décapage (Figure 3), ce n'est pas le cas du glycérol (Figure 2). Ainsi, l'effet de décapage est sensiblement le même pour le DMSO pur (Figure 1) et le mélange DMSO + glycérol (90/10 p/p).

### Exemple 2 : Préparation d'une composition décapante DMSO glycérolé + MEK

On a formulé le DMSO glycérolé avec de la MEK dans les proportions suivantes :
- DMSO glycérolé : 30% en poids ; et
- MEK : 70% en poids.

On a préparé une formulation de référence :
- DMSO : 30% en poids ; et
- MEK : 70% en poids.

On a conduit le même essai de décapage qu'à l'Exemple 1 avec chacune de ces deux formulations.

Les résultats visuels de ces deux essais peuvent être observés sur les Figures 4 et 5. L'effet est le même dans les deux cas, à savoir la formation d'écailles décollées du support, signe d'une bonne efficacité du décapage. Il est ainsi confirmé que le glycérol n'a pas d'effet d'inhibition du décapage.

## Revendications

1. -Utilisation d'un triol comme additif au diméthylsulfoxyde (DMSO) pour abaisser le point de cristallisation de ce dernier, **caractérisée par le fait que** l'additif au DMSO est le glycérol.

2. -Utilisation selon l'une des revendications 1 à **caractérisée par le fait qu'**au triol est ajoutée de l'eau, auquel cas du chlorure de sodium peut également être ajouté.

3. -Utilisation selon l'une des revendications 1 à 2, **caractérisée par le fait que** l'additif au DMSO est constitué, pour 100 parties en poids, par
(A) 75 à 100 parties en poids de triol;
(B) 0 à 15 parties en poids d'eau ;et
(C) 0 à 10 parties en poids de chlorure de sodium, lequel ne peut être présent que si de l'eau est présente.

4. -Utilisation selon l'une des revendications 1 à 3, **caractérisée par le fait que** l'additif constitué par le triol avec éventuellement l'eau et le cas échéant le chlorure de sodium représente 5 à 40 parties en poids, en particulier 10 à 30 parties en poids, pour 100 parties en poids de DMSO additivé.

## Patentansprüche

1. Verwendung eines Triols als Additiv zu Dimethylsulfoxid (DMSO), um den Kristallisationspunkt des letzteren zu senken, **gekennzeichnet dadurch, dass** das DMSO-Additiv Glycerol ist.

2. Verwendung nach einem der Ansprüche 1 bis 1, **dadurch gekennzeichnet, dass** dem Triol Wasser zugesetzt wird, wobei in diesem Fall Natriumchlorid ebenfalls zugesetzt werden kann.

3. Verwendung nach einem der Ansprüche 1 bis 2, **dadurch gekennzeichnet, dass** das Additiv zu DMSO, bezogen auf 100 Gewichtsteile, besteht aus:
(A) 75 bis 100 Gewichtsteile Triol;
(B) 0 a 15 Gewichtsteile Wasser; und
(C) 0 bis 10 Gewichtsteile Natriumchlorid, welches nur vorhanden sein kann, wenn Wasser vorhanden ist.

4. Verwendung nach einem der Ansprüche 1 bis 3, **dadurch gekennzeichnet, dass** das Additiv, das aus Triol mit eventuell Wasser und gegebenenfalls Natriumchlorid zusammengesetzt ist, 5 bis 40 % Gewichtsteile darstellt, insbesondere 10 bis 30 Gewichtsteile, auf 100 Gewichtsteile DMSO und Additiv.

## Claims

1. Use of a triol as an additive to dimethyl sulfoxide (DMSO), to lower the crystallization point of the latter, **characterized by** the fact that the additive to DMSO is glycerol.

2. Use according to any one of claims 1 to 1, **characterized in that** water is added to the triol, in which case sodium chloride can also be added.

3. Use according to any one of claims 1 to 2, **characterized in that** the additive to DMSO comprises, per 100 parts by weight,
(A) 75 to 100 parts by weight of triol;
(B) 0 to 15 parts by weight of water; and
(C) 0 to 10 parts by weight of sodium chloride, which can only be present if water is present.

4. Use according to any one of claims 1 to 3, **characterized in that** the additive, comprised by the triol with optionally water and, if necessary, sodium chloride, represents 5 to 40 parts by weight, in particular 10 to 30 parts by weight, per 100 parts by weight of DMSO and additive.
